# EUROPEAN PATENT SPECIFICATION

(11) **EP 3 220 838 B1**
(45) Date of publication and mention of the grant of the patent: **23.10.2019**
(21) Application number: 15801966.1
(22) Date of filing: 17.11.2015
(51) Int. Cl.: A61B 17/34, A61B 90/50

(54) **PROXIMAL-END SECUREMENT OF A MINIMALLY INVASIVE WORKING CHANNEL**
SICHERUNG EINES PROXIMALEN ENDES EINES MINIMAL INVASIVEN ARBEITSKANALS
FIXATION DE L'EXTRÉMITÉ PROXIMALE D'UN CANAL DE TRAVAIL À INVASIVITÉ MINIMALE

(30) Priority: 18.11.2014 US 201414546620
(43) Date of publication of application: 27.09.2017
(73) Proprietor: Medos International S.à.r.l, 2400 Le Locle (CH)
(72) Inventor: CHEGINI, Salman, CH-3004 Bern (CH); RICHTER, Joern, CH-4436 Oberdorf (CH); THOMMEN, Daniel, CH-4436 Oberdorf (CH); SENN, Peter, CH-4436 Oberdorf (CH)
(74) Representative: Orr, Robert
(86) International application number: PCT/US2015/060978
(87) International publication number: WO 2016/081403

(56) References cited:
- DE-U1- 29 916 026
- US-A- 5 080 662
- US-A- 5 820 623
- US-A1- 2011 028 791
- US-A1- 2014 066 940

## Description

### BACKGROUND OF THE INVENTION

The general trend in the treatment of the spinal pathologies is toward minimally invasive approaches to reduce the trauma on the surrounding tissues during the operation. For treatment of the lumbar spine pathologies, a percutaneous approach may be chosen within a working channel of 4-12 mm. The working channel serves as a safety barrier between the working instruments and the sensitive tissues (e.g. nerves and blood vessels) during the operation. The process of treatment including disc removal, endplate preparation, implant insertion and graft material insertion should be performed through the working channel.

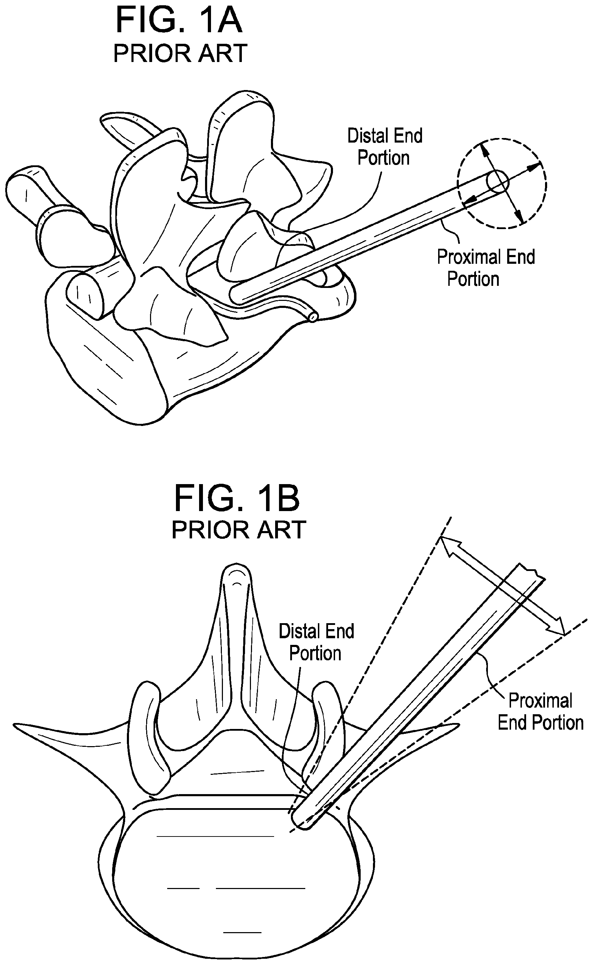

In order to ensure the safety of these procedures, the distal end portion of the working channel (from surgeon's perspective) should be secured/anchored onto desired points (see FIGS. 1A and 1B). Typically, these points are either bone or disc tissue. In addition to the fixation of the distal end portion of the working channel, depending on the procedure that is being performed, the proximal end portion of the working channel needs to be able to either move laterally, move cranially/caudally, or be substantially fixed. For example, during disc removal, the surgeon might want to change the angle of the working channel in order to gain better access to more of the remaining disc tissue (see FIG. IB). At the same time, it might be desired that this motion be limited to a given range, and to be fixed in the axial direction. Furthermore, in some instances, it might be desired for the proximal portion of the working channel to be fully fixed in order to create a two-point fixed channel between proximal and distal portion.

US-2011/0028791 A1 discloses an arcuate surgical guidance system. Minimally-invasive devices are used through small access portals to minimize trauma to structures adjacent the treatment site. An access device disclosed in the document includes a guide frame and a cannula support washer moveably coupled to the guide frame. The washer has a cannula port aperture extending therethrough and a surface geometry complementary to the surface geometry of the guide frame. The complementary surface geometries limit movement of the cannula port aperture to arcuate movement along a surface segment of a sphere. The guidance system includes a lower guide element, upper guide element, and an alignment washer having a central port through which a cannula can be inserted. The lower guide element, upper guide element and the alignment washer have a complementary geometry that conforms to a segment of a sphere. The two guide elements couple together with the alignment washer disposed in a space between them forming an arcuate guide for a cannula. The space between the guide elements is at least as wide as the alignment washer is thick, such that the washer can be manipulated in sliding fashion between the upper and lower guide elements.

DE-29916026 U1 discloses a holding device for a surgical instrument having a tubular shaft, in particular an optical imaging system comprising a tubular imaging element comprising holding means for the surgical instrument. The holding device is positioned above a surgical field, in particular over a trocar, via a holding structure which is fixed on a surgical table, for example, so that a tubular shaft of a surgical instrument can be inserted. The tube is then inserted and the surgical instrument inserted into a receptacle. For finer adjustment, a holding carriage can be displaced, relative to a center point, on a guide track in order to rotate the tube shaft in the trocar about an axis passing through the center point. By actuating a rotary knob, by means of a positioning element, an instrument holder and thus the surgical instrument can be displaced with its tube in a displacement direction transversely to a path plane.

### SUMMARY OF THE INVENTION

The present invention is directed at minimally invasive systems in which the proximal end portion of the working channel has either zero or a limited range of movement in the lateral direction.

Therefore, in accordance with the present invention, there is provided an apparatus as claimed in claim 1.

Optional further features of the apparatus are defined in the dependent claims.

A minimally -invasive surgical access system is also disclosed, which does not fall within the scope of the invention as claimed, the system comprising:
a) a tube having an outer wall, a longitudinal bore, a proximal end portion and a distal end portion;
b) a sliding tab comprising a collar having a pair of opposed flanges extending therefrom, wherein the collar is slidable along the outer wall of the tube; and
c) an annular frame having a pair of substantially opposed slots,
wherein the flanges of the collar respectively extend through the slots of the annular frame, and
wherein the tube extends through the annular frame.

Another minimally -invasive surgical access system is disclosed, which does not fall within the scope of the invention as claimed, the system comprising;
a) a tube having an outer wall, a longitudinal bore, a proximal end portion, a distal end portion, and a substantially spherical element radially surrounding a segment of the outer wall;
b) a sliding tab having a base and a pair of opposed flanges extending therefrom; the base having a hole therethrough defining a rim having a static portion and a slidable portion,
c) an annular frame having a pair of substantially opposed slots,
wherein each flange of the sliding tab extends through a respective slot of the annular frame,
wherein the tube extends through the annular frame, and
wherein the static portion of the rim releasably contacts a first portion of the substantially spherical element and the slidable portion of the rim releasably contacts a second portion of the substantially spherical element.

Another minimally invasive surgical access system is disclosed, which does not fall within the scope of the invention as claimed, the system comprising;
a) an upper cap describing a first portion of a substantially spherical surface,
b) an middle cap describing a second portion of the substantially spherical surface and having a central hole,
c) a lower cap describing a third portion of the substantially spherical surface,
d) a tube having an outer wall having an attachment portion, a longitudinal bore, a proximal end portion, a distal end portion,
wherein the upper cap and the lower cap are attached to and radially extend from the outer wall of the tube,
wherein at least one of the upper cap and the lower cap is removably attached to the outer wall of the tube,
wherein the tube is received in the central hole of the middle cap, and
wherein the middle cap is received between between the upper cap and the lower cap.

### DESCRIPTION OF THE FIGURES

FIGS. 1A and 1B disclose the desired ranges of motion for the systems of the present invention.
FIG. 2 discloses a first embodiment not of the present invention having a slidable collar.
FIG. 3 discloses a second embodiment not of the present invention having a substantially spherical element attached to the tube.
FIGS. 4A-B discloses a third embodiment not of the present invention having a plurality of caps.
FIGS. 5A-C disclose the different steps of mounting and securing the embodiment of FIG. 4.
FIG. 6A discloses an exploded view of the apparatus of the fourth embodiment and in accordance with the present invention.
FIG. 6B discloses an assembled view of the apparatus of the fourth embodiment of the present invention.
FIGS 6C, 6K, 6L, 6M, 6N and 6O disclose various desirable orientations of the fourth apparatus embodiment relative to a functional spinal unit.
FIGS. 6D-6E disclose the possible cranial-caudal tilt angles of the fourth embodiment.
FIGS. 6F-6G disclose the possible medial-lateral tilt angles of the fourth embodiment.
FIGS. 6H-6J disclose respective side, perspective and top views of the fourth embodiment apparatus.
FIGS. 6K-6O disclose various views of the apparatus in relation to the spine.
FIGS. 6P-6Q disclose views of the fourth embodiment apparatus wherein the medial-lateral bar runs parallel to the spine.

### DETAILED DESCRIPTION OF THE INVENTION

For the purposes of the present invention, the "distal end portion of the tube" is the portion of the tube that is nearest to the patient and furthest from the surgeon when the tube is inserted into the patient, and the "proximal end portion of the tube" is the portion of the tube that is furthest from the patient and nearest to the surgeon when the tube is inserted into the patient. Also, "a working channel" and "a tube" are considered to be interchangeable.

In the following description, several concepts are described covering the subjects of a) limiting lateral motion of the proximal end portion of the tube, b) eliminating the lateral motion of the proximal end portion of the tube, and c) eliminating the axial motion of the proximal end portion of the tube.

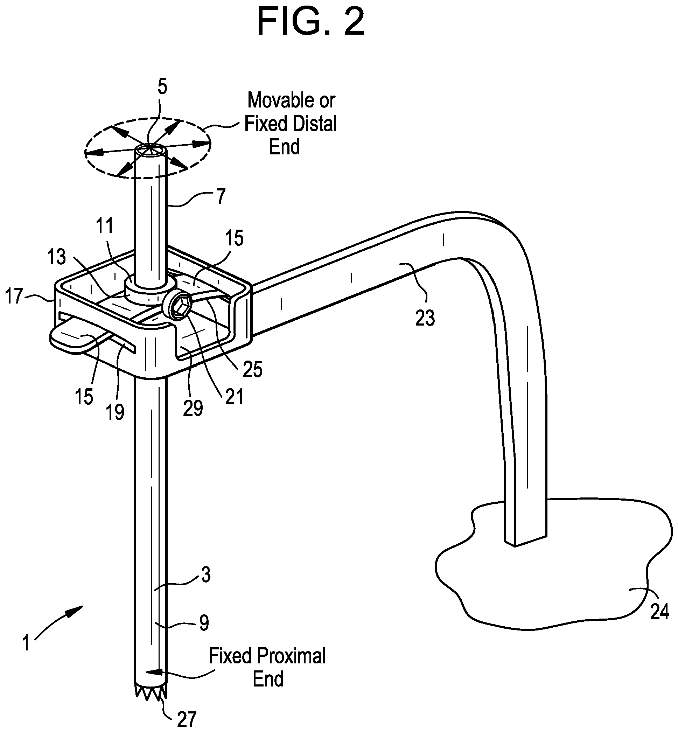

Now referring to FIG. 2, there is provided a minimally -invasive surgical access system, comprising;
a) a tube 1 having an outer wall 3, a longitudinal bore 5, a proximal end portion 7 and a distal end portion 9;
b) a sliding tab 11 comprising a collar 13 having a pair of opposed flanges 15 extending therefrom, wherein the collar is slidable along the outer wall of the tube; and
c) an annular frame 17 having a pair of substantially opposed slots 19,
wherein the flanges of the collar respectively extend through the slots of the annular frame, and
wherein the tube extends through the annular frame.

The embodiment shown in FIG. 2 includes an annular frame that can be fixed onto a stationary unit, such as the operating table, so as to anchor the system. As previously described, the distal end portion of the tube can be fixed onto the bony structures (such as a vertebral body) or soft tissues (such as disc cartilage) within the lumbar spine. When the tube is so distally anchored, the proximal end portion of the tube can move in a substantially conical volume, with the distal end of the tube being its apex. The fixed-in-space annular frame of the embodiment of FIG. 2 limits the range of the motion of the proximal end portion of the tube. The sliding tab component of the system is comprised of a collar with a pair of opposed flanges extending therefrom. Preferably, the shape of the flanges describes a portion of a spherical surface that mimics the limited motion of the tube. The outer annular frame has a pair of opposed matching slots that slidably receive their respective flanges. Preferably, each slot is shaped as an arc that matches the arc-like transverse cross-section of the flange it receives. The working channel is mounted and fixed onto the sliding tab using a set screw 21 that is received in a threaded hole in the collar. The set screw can extend through the collar and contact the outer wall of the tube in the proximal end portion of the tube so as to lock the collar to the tube, thereby preventing the motion of the tube channel in the axial direction. The limits of the lateral motion of the proximal end portion are defined by the outer annular frame. The outer annular frame can be fixed onto the operating table 24 using an extension arm 23.

Therefore, in preferred embodiments of the first embodiment, the system further comprises an arm extending from a stationary unit, wherein the arm is attached to the annular frame. Preferably, the collar comprises a threaded hole, and the system further comprises a set screw received in the threaded hole of the collar. Preferably, the set screw can extend through the collar and contact the outer wall of the tube in the proximal end portion of the tube to lock the collar to the tube. Preferably, each flange comprises a portion of a spherical surface 25 and each slot describes an arc, wherein the flange mates with the slot. Preferably, the distal end portion of the tube has a docking feature (such as a plurality of distally -extending teeth 27) adapted to dock to bone or cartilage. In some embodiments, the collar does not contact the annular frame. In some embodiments, the annular frame has a cutout 29 adapted to allow access by a screwdriver to the collar in order to tighten or loosen the set screw. Preferably, this cutout aligns radially with the set screw. Preferably, the proximal end portion of the tube is able to move in a substantially frustoconical volume when the distal end portion of the tube is fixed.

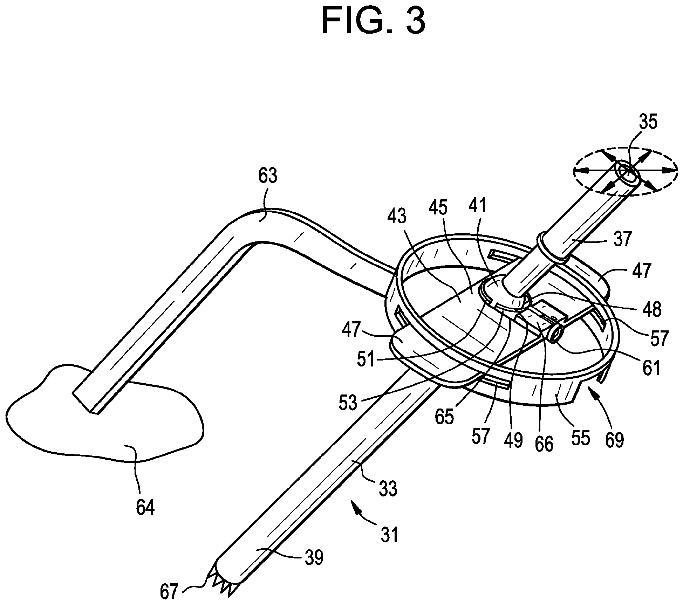

Now referring to FIG. 3, there is provided a minimally -invasive surgical access system, comprising;
a) a tube 31 having an outer wall 33, a longitudinal bore 35, a proximal end portion 37, a distal end portion 39, and a substantially spherical element 41 radially surrounding a segment of the outer wall;
b) a sliding tab 43 having a base 45 and a pair of opposed flanges 47 extending therefrom; the base having a hole 48 therethrough defining a rim 49 having a static portion 51 and a slidable portion 53,
c) an annular frame 55 having a pair of substantially opposed slots 57,
wherein each flange of the sliding tab extends through a respective slot of the annular frame,
wherein the tube extends through the annular frame, and
wherein the static portion of the rim releasably contacts a first portion of the substantially spherical element and the slidable portion of the rim releasably contacts a second portion of the substantially spherical element.

The second embodiment of FIG. 3 includes an outer annular frame that could be fixed onto a stationary unit, such as the operating table. As previously described, the distal end portion of the tube can be fixed onto the bony structures or soft tissues of the spine, so that the proximal end portion of the tube can move in a substantially frustoconical volume with the distal tip being the apex. In this particular embodiment, the sliding tab has flat flanges, which are easier to manufacture. Likewise, the outer annular frame has a pair of simple, linear slots to slidably receive the flanges. The sliding tab has an axial hole therein defining a rim, the rim comprising a static hemispherical portion, as well as a movable hemispherical portion. As the working channel passes into the sliding-tab, the set screw 61 can be turned to move the dynamic hemisphere to hold or release the spherical protrusion of the working channel, thereby fixing or release the angel of the sliding tab with respect to the tube. This allows for the movement on the desired range. The whole structure allows for sideways motion of the distal end in a given range (defined by the slot on the outer frame) and blocks the axial motion of the distal end.

Preferably, in this second embodiment, the system further comprises an arm 63 extending from a stationary unit 64, wherein the arm is attached to the annular frame. Preferably, the base comprises a first cutout 65, and further comprises a sliding door 66 slidably received in the cutout. Preferably, the sliding door comprises the second portion of the rim. Preferably, the sliding door further comprises a substantially hemispherical portion extending from the slidable portion of the rim, wherein the substantially hemispherical portion releasably contacts the second portion of the substantially spherical element to lock the sliding tab to the tube. Preferably, the sliding door is slidably actuated by a set screw. Preferably, each flange of the sliding tab is flat and each respective slot is substantially rectangular, so that the flange mates with the slot. Preferably, the distal end portion of the tube has a docking feature (such as distally extending teeth 67) adapted to dock to bone or cartilage. In some embodiments, the substantially spherical element does not contact the annular frame. Preferably, the annular frame has a second cutout 69 (designed to allow access by a screwdriver) that aligns radially with the set screw. Preferably, the proximal end portion of the tube is able to move in a substantially frustoconical volume when the distal end portion of the tube is fixed. In some embodiments, the flat flanges of the sliding tab are not orthogonal to the tube.

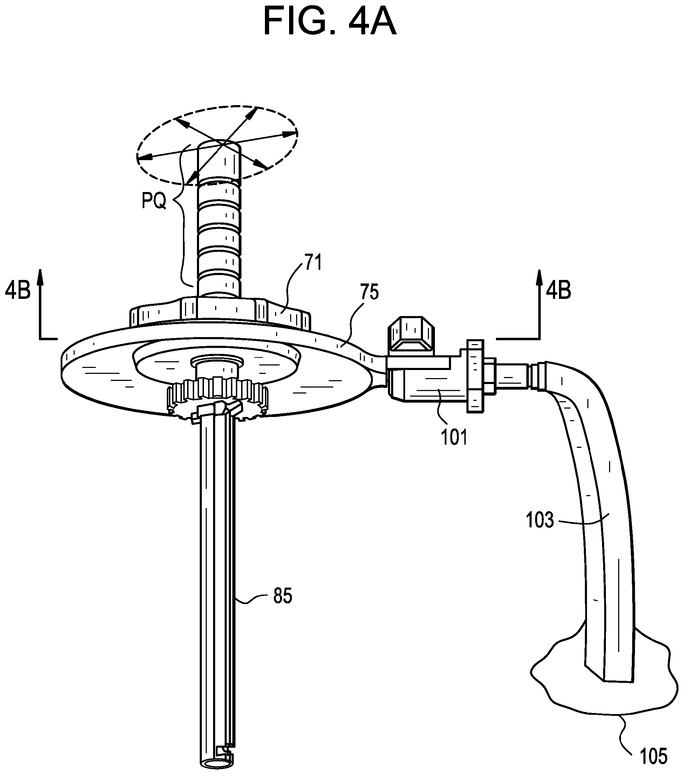

Now referring to FIGS. 4A -4B, there is provided a minimally invasive surgical access system, comprising;
a) an upper cap 71 describing a first portion 73 of a substantially spherical surface,
b) an middle cap 75 describing a second portion 77 of the substantially spherical surface and having a central hole 79,
c) a lower cap 81 describing a third portion 83 of the substantially spherical surface,
d) a tube 85 having an outer wall 87 having an attachment portion 89, a longitudinal bore 91, a proximal end portion 93, a distal end portion 95,
wherein the upper cap and the lower cap are attached to and radially extend from the outer wall of the tube,
wherein at least one of the upper cap and the lower cap is removably attached to the outer wall of the tube,
wherein the tube is received in the central hole of the middle cap, and
wherein the middle cap is received between between the upper cap and the lower cap.

This concept comprises three spherical caps on top of each other. The middle cap is the proximal point where the rigid arm is fixed. The lower cap extends from the working channel and is preferably integral with the working channel. This lower cap helps to prevent the working channel from being pulled proximally through the hole of the middle cap. The middle cap has a hole of predetermined size that allows for limited lateral motion of the working channel, thereby defining the boundaries of allowed motion. The middle cap is fixed to the operating table via attachments as described above. This middle cap may have a fixation element to help with the fixation. The upper cap has a threaded hole 97 and, when threaded onto the threaded 89 portion of the working channel, helps preventing the channel from advancing distally. In this concept, if the upper cap is advanced distally, it will create friction between the caps and will prevent the motion of the caps relative to each other. In other words, this concept allows for the motion of the working channel and at the same time allows for complete fixation of the distal and proximal ends of the working channel at desired direction.

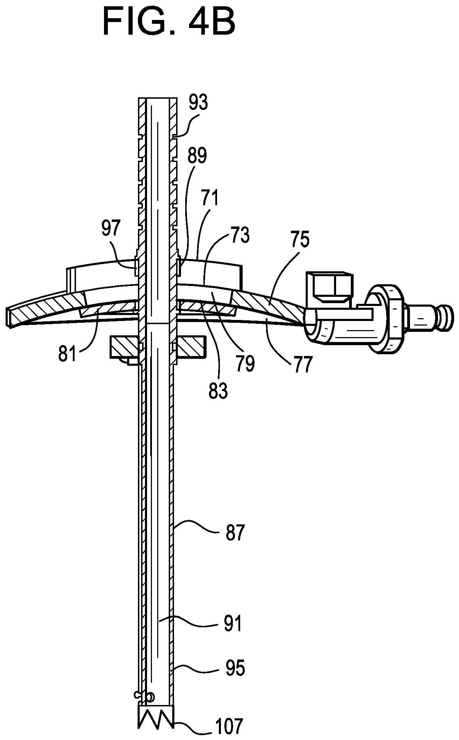

Preferably, in the embodiment of FIG. 4, the upper cap is located proximal to the lower cap. Preferably, the middle cap has a fixation element 101 for fixation to a stationary unit. Preferably, the system further comprises an arm 103 extending from a stationary unit 105, wherein the arm is attached to the fixation element. Preferably, the proximal portion of the tube is able to move in a substantially frustoconical volume when the distal end portion of the tube is fixed. Preferably, the distal end portion of the tube has a docking feature 107 adapted to dock to bone. Preferably, the upper cap, middle cap, and lower cap are located in a proximal-most quarter PQ of the tube.

In some embodiments, the upper cap has a threaded hole 97, the outer wall of the working channel has a threaded portion 89 , and wherein the upper cap is threadably received on the threaded portion of the outer wall of the tube.

In FIG. 4, the upper cap is shown to be removable by virtue of its threadability upon the outer wall of the tube. However, removability is not restricted to threaded features. For example, in some embodiments, the tube and cap may provide a Morse taper lock. In other embodiments, the cap is made of an elastic material that snugly fits the outer wall of the tube.

In some embodiments, one of the upper cap and the lower cap is removably attached to the outer wall of the tube, and the other is integrally attached to the outer wall of the tube.

In some embodiments, one of the upper or lower cap has a threaded hole, the outer wall has a threaded portion, and the cap having the threaded hole is threadably received on the threaded portion of the outer wall of the tube.

In some embodiments, both of the upper cap and the lower cap are removably attached to the outer wall of the tube, preferably threadably attached.

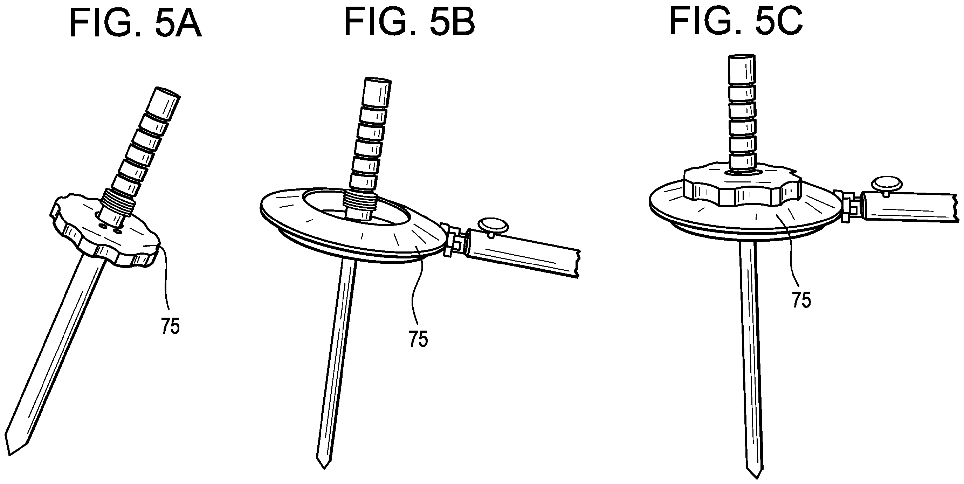

A functional prototype of this method is shown in FIGS. 5A-C, with different steps of mounting and securing. In FIG. 5A, a tube having an upper threaded portion and a lower cap permanently attached thereto is provided. In FIG. 5B, the middle cap is lowered onto the lower cap. In FIG. 5C, an upper cap with a threaded hole is placed over the middle cap and threaded onto the threaded portion of the tube, thereby trapping the middle cap between the upper and lower caps. Lastly, the middle cap is affixed to a stationary unit.

In some embodiments, the features of the upper and lower caps are reversed. Therefore, in accordance with the present invention, one of the upper cap and the lower cap is removably attached to the outer wall of the tube, and the other of the caps is integrally attached to the outer wall of the tube. Alternatively, both of the upper cap and the lower cap are removably attached to the outer wall of the tube.

It is believed that the above-described embodiments are generally suitable for use in typical percutaneous spinal surgeries, in conjunction with working channel diameters of only a few millimeters. However, there are certain spinal surgeries in which use of the above embodiments could require very large and bulky constructs. These certain surgeries (which include direct decompression surgeries performed through a mini-open posterior or para-medial approach) often require:
a) the tube of the working channel diameter to be larger-than-usual (e.g., from about 10 mm to about 30 mm in diameter), or
b) larger cranial-caudal and medial-lateral tilt angles, so that a larger angular range of motion is realized.

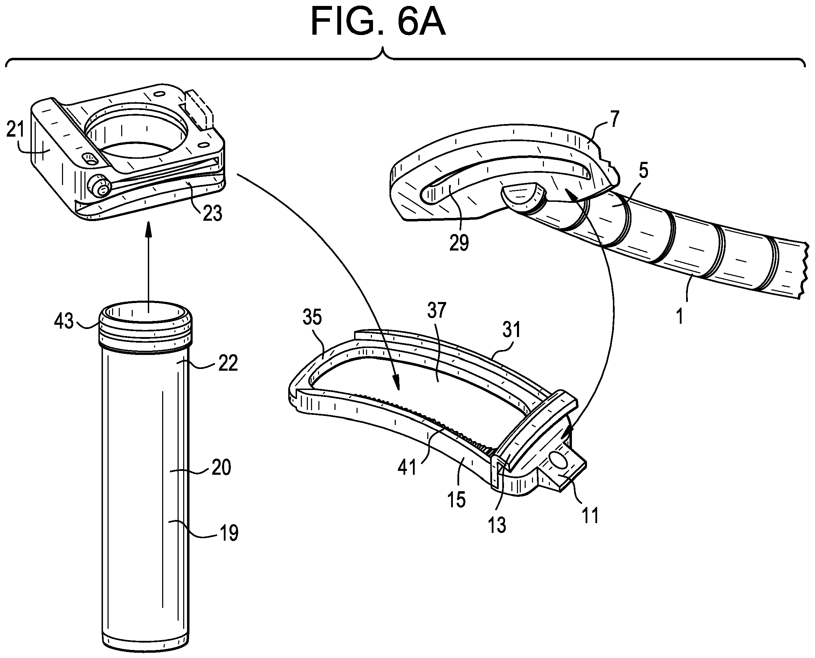

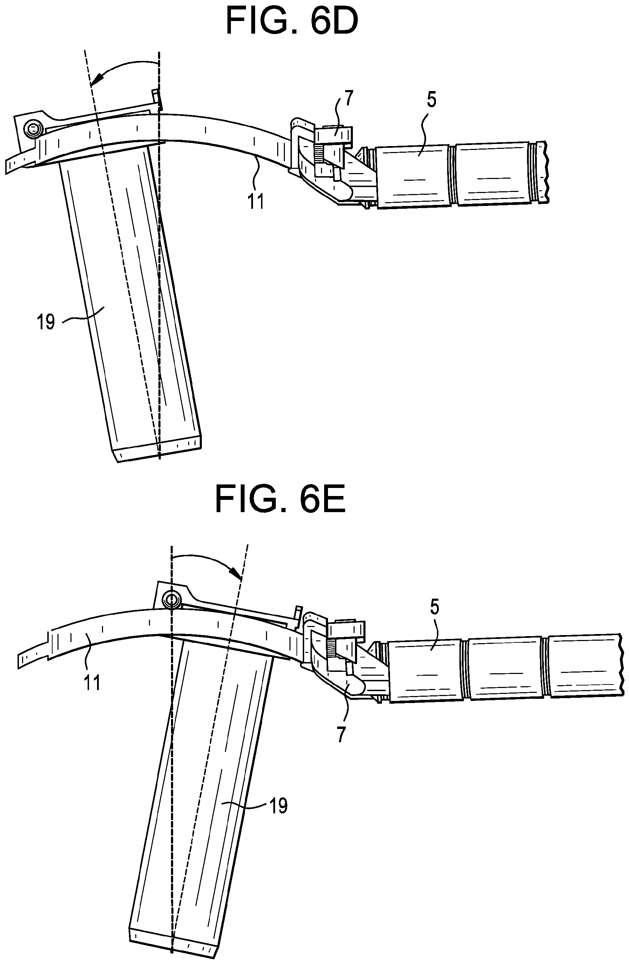

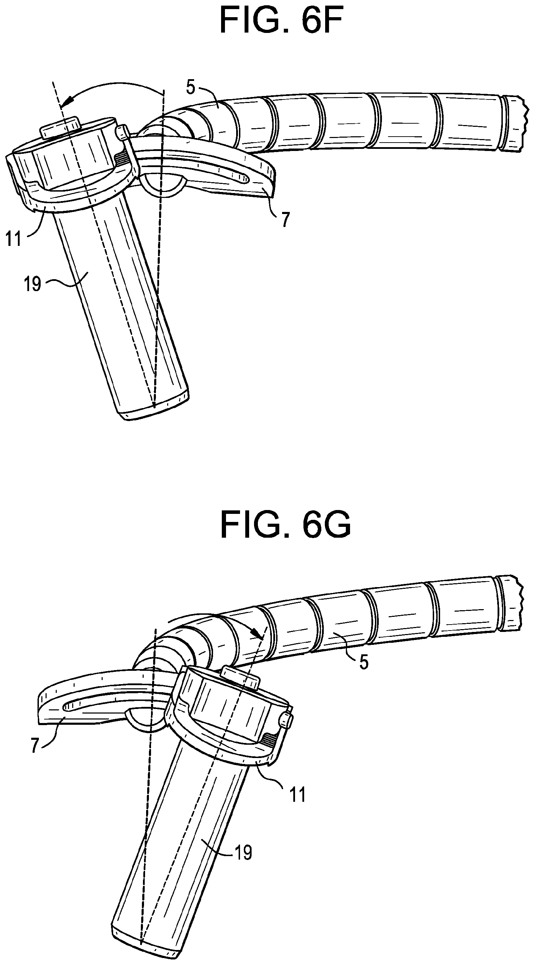

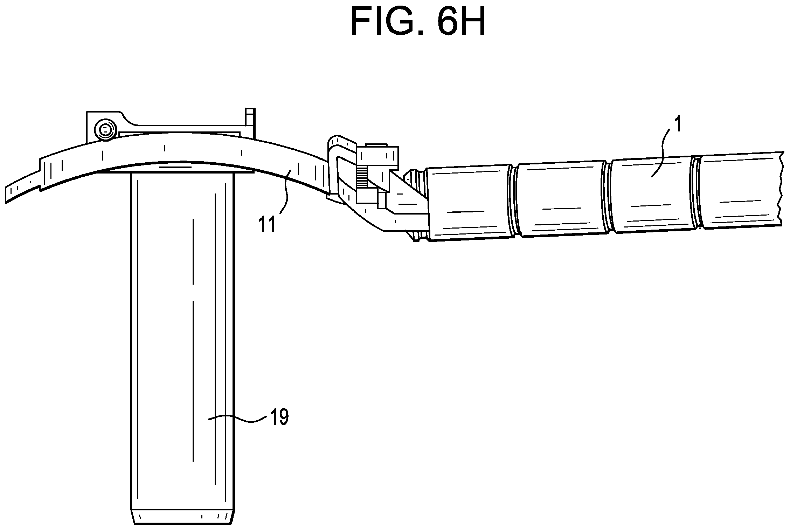

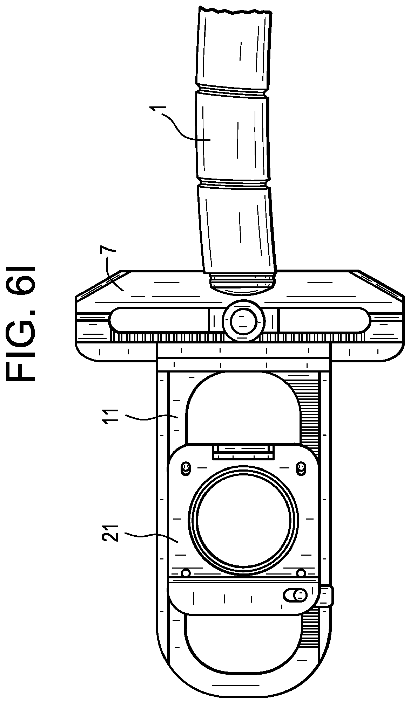

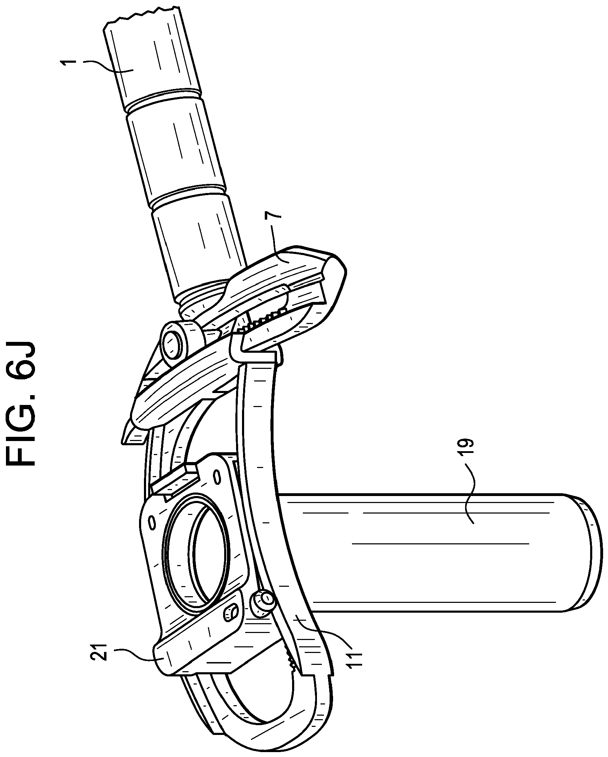

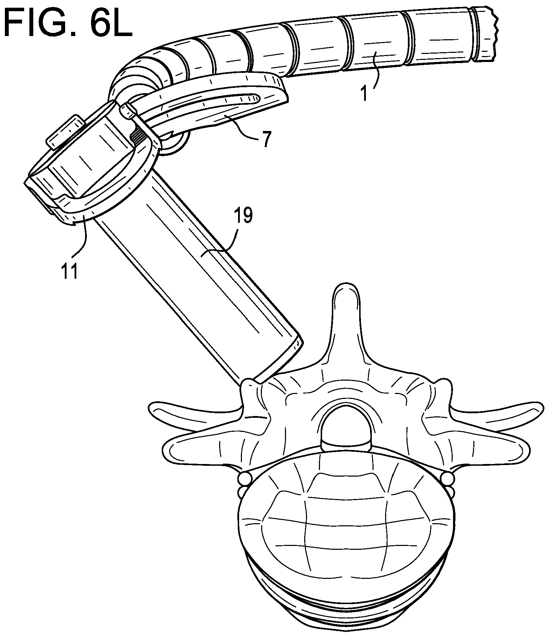

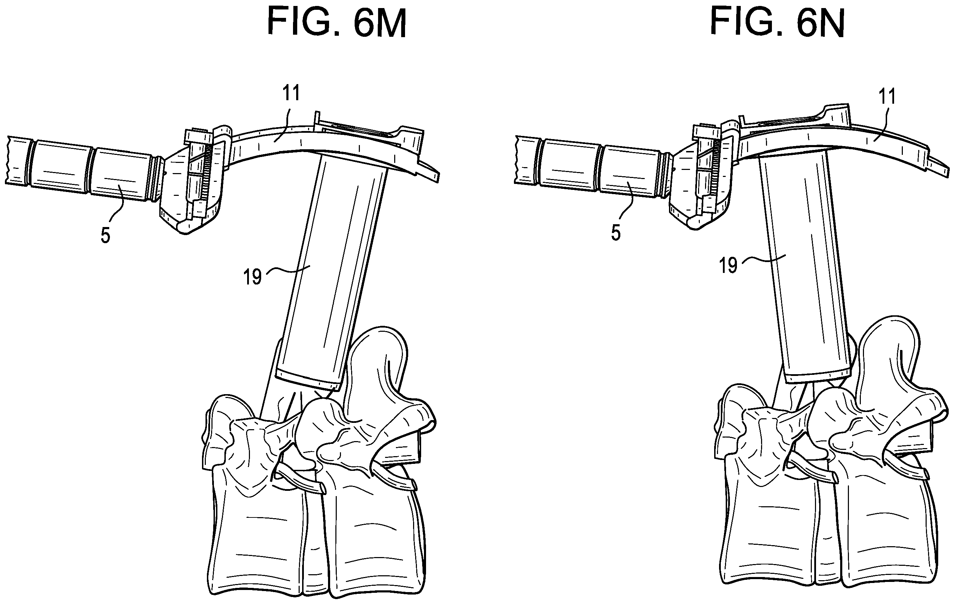

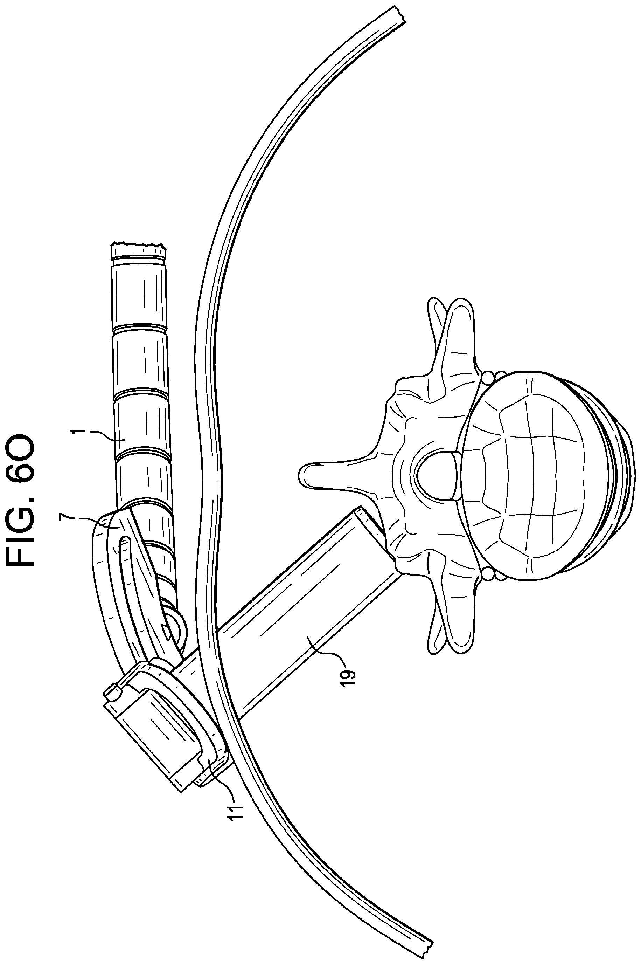

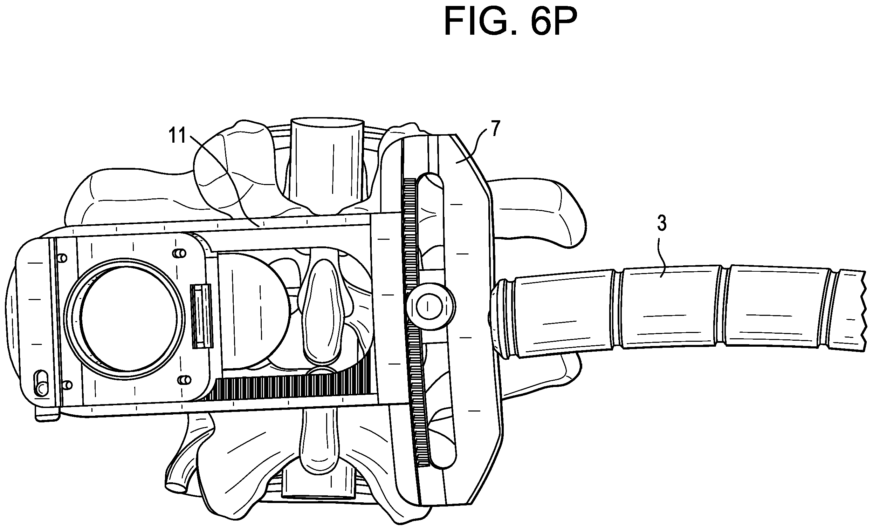

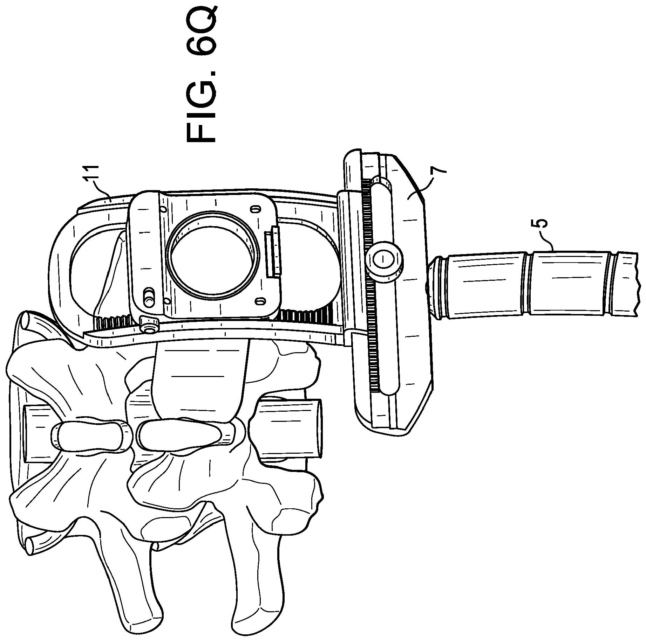

Therefore, in an effort to address these situations, in a fourth embodiment, in accordance with the present invention, and now referring to FIGS. 6A-6O, there is provided an apparatus comprising:
a) an arm 1 having a proximal end portion 3 connected to a stationary object and a distal end portion 5,
b) a medial-lateral bar 7 connected to the distal end portion of the arm and having a first rail 9;
c) a cranial-caudal bar 11 having:
   i) a first rail 13 in slidable engagement with the first rail of the medial-lateral bar in a first direction and
   ii) a second rail 15 extending substantially perpendicularly from the first rail of the cranial-caudal bar;
d) a working channel construct 17 comprising:
   i) a tube 19 having an outer surface 20 and a proximal end portion 22, and
   ii) a slider 21 attached to the outer surface of the tube and having a first rail 23 in slidable engagement with the second rail of the cranial-caudal bar.

This fourth embodiment functions substantially similarly to the previously-described embodiments. For example, its working channel tube has a restricted range of motion in the axial direction. Secondly, the fourth embodiment also allows for angular movement of the proximal end of the working channel construct, so as to always leave the tube's tip in the same position. See, for example, FIGS. 6D-6G.

This fourth embodiment is especially suitable in direct decompression surgeries when a) the tube of the working channel diameter needs to be from about 10 mm to about 30 mm in diameter, or b) larger cranial-caudal and medial-lateral tilt angles are needed, so that a larger angular range of motion is needed.

In FIG. 6A-6B of the present invention, during the surgery, the working channel tube may be attached to and detached from a slider. Various coupling or push- button mechanisms can be selected for this attachment.

In FIGS. 6A-6O, the slider is slidably connected to the cranial-caudal bar by mating rails. Preferably, the mating rails of the slider and cranial-caudal bar have mating arcuate shapes. See FIGS. 6D-6E. Preferably, the curvature of these arcuate rails is selected so that the common radius defined by their curves corresponds to the distance between the curve location and the tip of the working channel tube in the final assembly. In other words, the distal tip of the working channel tube defines the centerpoint of the circle described by the mating rails. In this way, it is insured that when the position of the slider along the rail of the cranial-caudal bar is changed in space, the location in space of the centerpoint tube distal end does not change, only the direction of the working channel tube central axis changes. Typically, the curved slider rail can smoothly glide along its mating curved rail of the cranial-caudal bar (thereby continuously changing the cranial-caudal angle of the working channel construct). However, in some embodiments, the relative positions of these rails against each other can be fixed. Various mechanisms can be selected to fix a relative position of these rails. For example, opposing teeth can be provided along each of the mating rails, as in FIG. 6A. These teeth can act as anchors when a fixation is desired. This fixation defines the cranial-caudal angle of the working channel tube.

In FIGS. 6A-6O, the first and second rails of the cranial-caudal bar are each arcuate. Preferably, the arcs of these rails are equal, so that the cranial-caudal bar defines a spherical surface. The center of the sphere is defined by the tip location of the working channel construct in the final assembly. The rail of the medial-lateral bar mates with the first rail of the cranial-caudal bar and so also preferably has an arcuate shape of the same radius. The two bar components are slidably connected to each other by these arcuate rails. A bolt-slot connection or similar construct can be used to slidably connect the two bars in order to assure that the cranial-caudal bar not only keeps its perpendicular orientation relative to the medial-lateral bar, but is also able to slide along the slot. The position of the cranial-caudal bar relative to the medial-lateral bar defines the medial-lateral angle of the working channel construct. The position can be fixed if desired. Various mechanisms can be considered to realize the position of fixation. For example, teeth can be provided along each rail that act as anchors.

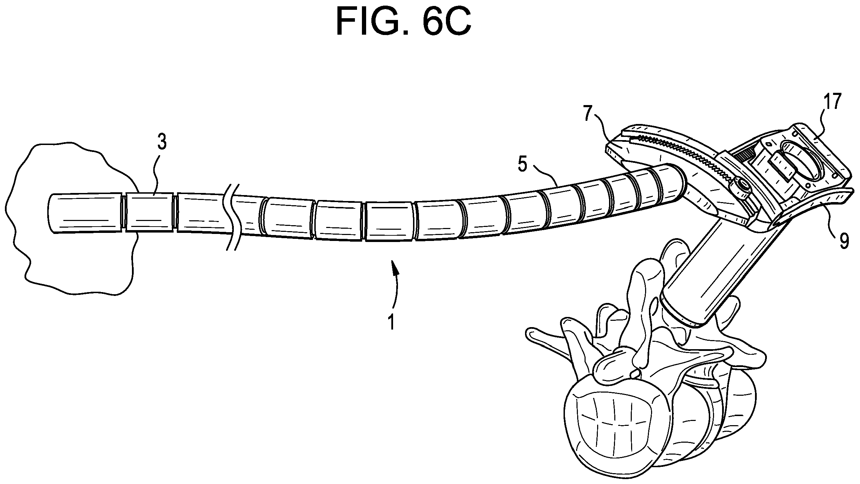

In FIG. 6C, the medial-lateral bar is attached to a rigid arm, whose position can be fixed relative to the operating room table during the surgery.

Although the cranial -caudal bar is shown in FIG. 6A as having four rails forming a rectangle with an internal window, in some embodiments, the cranial-caudal bar could potentially consist of 3 rails (defining a "U"-Shape) or 2 rails (defining an "L-"shape). Although the described "4-rail" configuration likely provides for the highest stability of these embodiments against bending of the working channel construct around the axis along the bar, and can provide for the finest dimensions of the rails at the same time, an very stiff sliding/connection mechanism that only needs support from one rail, or without the stabilization of the closing-rod, can also be considered as well.

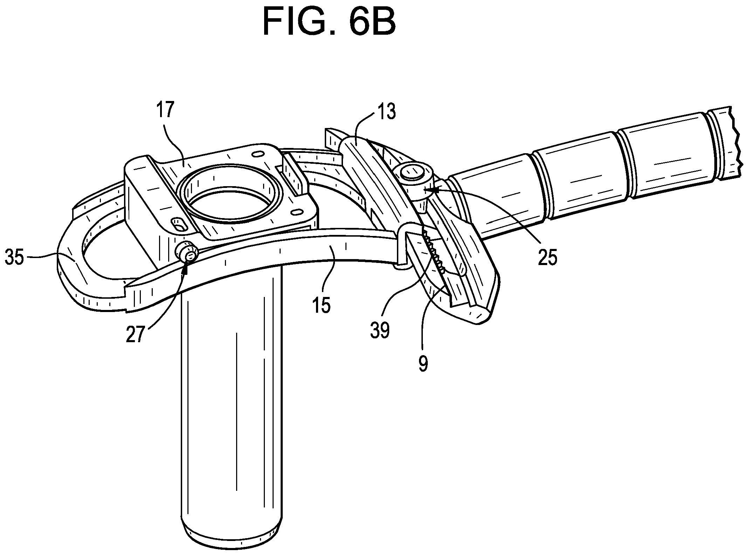

In some embodiments (as in FIG. 6B), the cranial-caudal bar has a release button 25 for releasable attachment to the medial-lateral bar. Typically, pressing this button releases the engagement of a teeth-like element on the cranial-caudal bar within the tee engages the teeth of the rail of the medial-lateral bar.

In some embodiments (as in FIG. 6B), the slider has a release button 27 for releasable attachment to the cranial-caudal bar. Typically, this button engages the teeth of the second rail of the cranial-caudal bar.

In some embodiments (as in FIG. 6A), the medial-lateral bar has a first window 29 or slot therein for slidable reception of the cranial-caudal bar. Typically, the slot is adjacent the rail of the medial-lateral bar, and the first rail of the cranial-caudal bar has a bolt-like shape so as to provide a slidable bolt-slot connection with the medial-lateral bar.

In some embodiments(as in FIG. 6A), the cranial-caudal bar has a third rail 31 extending from the first rail of the cranial-caudal bar in a direction substantially parallel to the second rail of the cranial-caudal bar. This embodiment can provide a cranial-caudal bar having a U-shape. In this embodiment, stops can be provided at the two ends of the U-shape so that the slider remains within the pocket of the U-shape.

In some embodiments, the slider further comprises iii) a second rail (not shown) substantially parallel to the first rail of the slider, wherein the second rail of the slider is in slidable engagement with the third rail of the cranial-caudal bar.

In some embodiments (as in FIG. 6B), the tube is disposed between the second and third rails of the cranial-caudal bar. This allows the forces that act upon the tube to be evenly supported by the pair of rails of the cranial-caudal bar.

In some embodiments (as in FIG. 6A), a fourth rail (connecting bar) 35 connects the second and third rails of the cranial-caudal bar to form a second window 37, and the tube extends through the second window. This ensures that the slider will remain slidably attached to the cranial-caudal bar.

In some embodiments (as in FIG. 6B), the first rail of the medial-lateral bar and the first rail of the cranial-caudal bar have mating teeth 39 thereon, and the second rail of the cranial-caudal bar and the first rail of the working channel construct have mating teeth 41 thereon. With the help of a member that is integrated to the component that is sliding along the rail with the teeth, and that can engage the teeth, this allows the relative positions of the components to be fixed, thereby insuring the location of the working channel tube relative to the patient.

In some embodiments, a medical device is located within the tube. In some embodiments, thereof, the medical device is an instrument, while in others the medical device is an implant. Typically, the medical device is passed from the proximal end portion of the tube to the distal end portion of the working tube.

In some embodiments, the first rail of the medial-lateral bar and the first rail of the cranial-caudal bar have matching arcuate shapes. This allows the tube to be tilted with respect to the patient in a first plane while maintaining the location of the distal end of the tube.

In some embodiments, the second rail of the cranial-caudal bar and the first rail of the slider have matching arcuate shapes. This allows the tube to be tilted with respect to the patient in a second plane while maintaining the location of the distal end of the tube.

In some embodiments, the slider is attachable and detachable from/ to the outer surface of the tube at the proximal end portion of the tube. This allows a fine control of the location of the proximal end portion of the tube. This allows as well that the tube can be introduced into the patient at the right location in a first step of a surgery. The rest of the components are pre-assembled and can be attached to the tube at this attachment location of the slider, while the arm is in a flexible configuration. After attaching the tube to the rest of the assembly, the arm can be brought to a rigid configuration, leaving only the option of changing the position of the tube, with respect to the patient to the angular changes by the rail connections.

In some embodiments, the first rail of the cranial-caudal is in slidable engagement with the first rail of the medial-lateral bar by virtue of a bolt-slot connection. This arrangement helps maintain the orientation of the cranial-caudal bar vis-a-vis the medial-lateral bar.

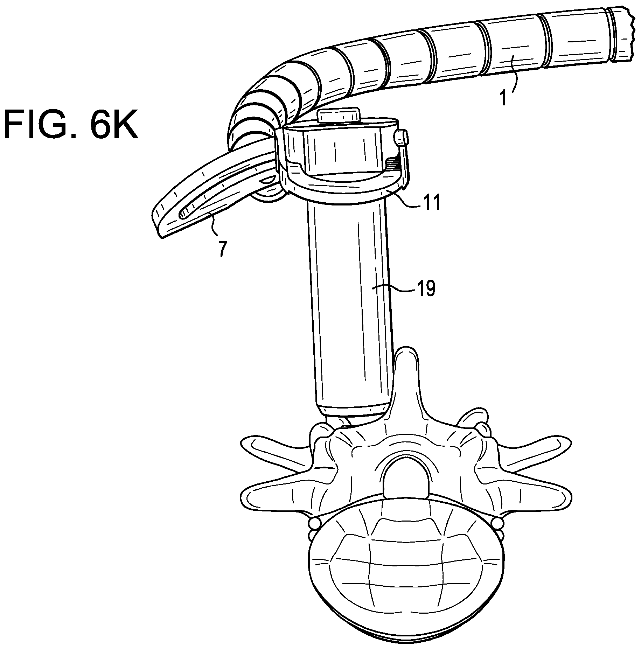

FIGS. 6K-6L disclose views of the fourth embodiment apparatus wherein the medial-lateral bar runs parallel to the spine.

The components of the present invention are preferably made from a biocompatible metal such as stainless steel, titanium alloy or cobalt-chrome. However, it is contemplated that the components can be made from polymeric materials so as to provide an inexpensive, single use system.

## Claims

1. A medical apparatus comprising:
an arm (1) having a proximal end portion (3) connected to a stationary object and a distal end portion (5),
a medial-lateral bar (7) connected to the distal end portion (5) of the arm (1) and having a first arcuate rail (9);
a cranial-caudal bar (11) having:
a first arcuate rail (13) in slidable engagement with the first arcuate rail (9) of the medial-lateral bar (7) in a first direction, wherein the first arcuate rail of the cranial-caudal bar and the first arcuate rail of the medial lateral-bar have matching arcuate shapes, and
a second arcuate rail (15) extending substantially perpendicularly from the first arcuate rail (13) of the cranial-caudal bar;
a working channel construct (17) comprising:
a tube (19) having an outer surface (20), a proximal end portion (22) and a distal end, and
a slider (21) attached to the outer surface (20) of the tube (19) and having a first arcuate rail (23) in slidable engagement with the second arcuate rail (15) of the cranial-caudal bar (11), wherein the first arcuate rail of the slider and the second arcuate rail of the cranial-caudal bar have matching arcuate shapes;
wherein the matching arcuate shapes of the first arcuate rail (13) of the cranial-caudal bar (11) and the first arcuate rail (9) of the medial lateral-bar (7) allow the tube (19) to be tilted in a first plane, to define a medial-lateral tilt angle of the tube, while maintaining a location of the distal end of the tube; and
wherein the matching arcuate shapes of the first arcuate rail (23) of the slider (21) and the second arcuate rail (15) of the cranial-caudal bar (11) allow the tube (19) to be tilted in a second plane, to define a cranial-caudal tilt angle of the tube, while maintaining the location of the distal end of the tube.

2. The apparatus of claim 1 wherein the cranial-caudal bar (11) has a release button (25) for releasable attachment to the medial-lateral bar (7).

3. The apparatus of claim 1 wherein the slider (21) has a release mechanism (27) for releasable attachment to the cranial-caudal bar (11).

4. The apparatus of claim 1 wherein the medial-lateral bar (7) has a first window (29) therein for slidable reception of the cranial-caudal bar (11).

5. The apparatus of claim 1 wherein the cranial-caudal bar (11) has a third rail (31) extending from the first rail (13) of the cranial-caudal bar (11) in a direction substantially parallel to the second rail (15) of the cranial-caudal bar.

6. The apparatus of claim 5 wherein the slider (21) further comprises a second rail substantially parallel to the first rail (23) of the slider, wherein the second rail of the slider is in slidable engagement with the third rail (31) of the cranial-caudal bar (11).

7. The apparatus of claim 6 wherein the tube (19) is disposed between the second and third rails (15, 31) of the cranial-caudal bar (11).

8. The apparatus of claim 7 wherein a fourth rail (35) connects the second and third rails (15, 31) of the cranial-caudal bar (11) to form a second window (37), and the tube (19) extends through the second window.

9. The apparatus of claim 1 wherein the first rail (9) of the medial-lateral bar (7) and the first rail (13) of the cranial-caudal bar (11) have mating teeth (39) thereon.

10. The apparatus of claim 1 wherein the second rail (15) of the cranial-caudal bar (11) and the first rail (23) of the slider (21) have mating teeth (41) thereon.

11. The apparatus of claim 1 further comprising:
a medical device located within the tube.

12. The apparatus of claim 11 wherein the medical device is an instrument or an implant.

13. The apparatus of claim 1 wherein the slider (21) is attached to the outer surface (20) of the tube (19) at the proximal end portion (22) of the tube.

14. The apparatus of claim 1 wherein the outer surface (20) of the tube (19) at the proximal end portion (22) of the tube has a thread thereon, the slider (23) has a window having a matching thread thereon, and the slider is threadably engaged with the proximal end portion of the tube.

15. The apparatus of claim 1 wherein the first rail (13) of the cranial-caudal bar (11) is in slidable engagement with the first rail (9) of the medial-lateral bar (7) by virtue of a bolt-slot connection.

## Patentansprüche

1. Medizinische Vorrichtung, umfassend:
einen Arm (1) mit einem proximalen Endabschnitt (3), der mit einem stationären Objekt verbunden ist, und einem distalen Endabschnitt (5),
eine Medial-Lateral-Stange (7), die mit dem distalen Endabschnitt (5) des Arms (1) verbunden ist und eine erste bogenförmige Schiene (9) aufweist;
eine Kranial-Kaudal-Stange (11) mit:
einer ersten bogenförmigen Schiene (13) in gleitendem Eingriff mit der ersten bogenförmigen Schiene (9) der Medial-Lateral-Stange (7) in einer ersten Richtung, wobei die erste bogenförmige Schiene der Kranial-Kaudal-Stange und die erste bogenförmige Schiene der Medial-Lateral-Stange übereinstimmende bogenförmige Formen aufweisen, und
einer zweiten bogenförmigen Schiene (15), die sich im Wesentlichen senkrecht von der ersten bogenförmigen Schiene (13) des Kranial-Kaudal-Stange erstreckt;
ein Arbeitskanal-Konstrukt (17), umfassend:
ein Rohr (19) mit einer Außenfläche (20), einem proximalen Endabschnitt (22) und einem distalen Ende, und
einen Schieber (21), der an der Außenfläche (20) des Rohrs (19) angebracht ist und eine erste bogenförmige Schiene (23) in gleitendem Eingriff mit der zweiten bogenförmigen Schiene (15) der Kranial-Kaudal-Stange (11) aufweist, wobei die erste bogenförmige Schiene des Schiebers und die zweite bogenförmige Schiene der Kranial-Kaudal-Stange übereinstimmende bogenförmige Formen aufweisen,
wobei die übereinstimmenden bogenförmigen Formen der ersten bogenförmigen Schiene (13) der Kranial-Kaudal-Stange (11) und der ersten bogenförmigen Schiene (9) der Medial-Lateral-Stange (7) ein Kippen des Rohrs (19) in einer erste Ebene ermöglichen, um einen medial-lateralen Neigungswinkel des Rohrs zu definieren, während eine Position des distalen Endes des Rohrs beibehalten wird; und
wobei die übereinstimmenden bogenförmigen Formen der ersten bogenförmigen Schiene (23) des Schiebers (21) und der zweiten bogenförmigen Schiene (15) der Kranial-Kaudal-Stange (11) ein Kippen des Rohrs (19) in einer zweiten Ebene ermöglichen, um einen kranial-kaudalen Neigungswinkel des Rohrs zu definieren, während die Position des distalen Endes des Rohrs beibehalten wird.

2. Vorrichtung nach Anspruch 1, wobei die Kranial-Kaudal-Stange (11) einen Freigabeknopf (25) zur lösbaren Anbringung an der Medial-Lateral-Stange (7) aufweist.

3. Vorrichtung nach Anspruch 1, wobei der Schieber (21) einen Freigabemechanismus (27) zur lösbaren Anbringung an der Kranial-Kaudal-Stange (11) aufweist.

4. Vorrichtung nach Anspruch 1, wobei die Medial-Lateral-Stange (7) in sich ein erstes Fenster (29) zur verschiebbaren Aufnahme der Kranial-Kaudal-Stange (11) aufweist.

5. Vorrichtung nach Anspruch 1, wobei die Kranial-Kaudal-Stange (11) eine dritte Schiene (31) aufweist, die sich von der ersten Schiene (13) der Kranial-Kaudal-Stange (11) in einer Richtung im Wesentlichen parallel zu der zweiten Schiene (15) der Kranial-Kaudal-Stange erstreckt.

6. Vorrichtung nach Anspruch 5, wobei der Schieber (21) ferner eine zweite Schiene im Wesentlichen parallel zu der ersten Schiene (23) des Schiebers umfasst, wobei die zweite Schiene des Schiebers in gleitendem Eingriff mit der dritten Schiene (31) der Kranial-Kaudal-Stange (11) ist.

7. Vorrichtung nach Anspruch 6, wobei das Rohr (19) zwischen der zweiten und der dritten Schiene (15, 31) der Kranial-Kaudal-Stange (11) angeordnet ist.

8. Vorrichtung nach Anspruch 7, wobei eine vierte Schiene (35) die zweite und die dritte Schiene (15, 31) der Kranial-Kaudal-Stange (11) verbindet, um ein zweites Fenster (37) zu bilden, und wobei sich das Rohr (19) durch das zweite Fenster erstreckt.

9. Vorrichtung nach Anspruch 1, wobei die erste Schiene (9) der Medial-Lateral-Stange (7) und die erste Schiene (13) der Kranial-Kaudal-Stange (11) zusammenpassende Zähne (39) an sich aufweisen.

10. Vorrichtung nach Anspruch 1, wobei die zweite Schiene (15) der Kranial-Kaudal-Stange (11) und die erste Schiene (23) des Schiebers (21) zusammenpassende Zähne (41) an sich aufweisen.

11. Vorrichtung nach Anspruch 1, ferner umfassend:
eine medizinische Vorrichtung, die sich in dem Rohr befindet.

12. Vorrichtung nach Anspruch 11, wobei die medizinische Vorrichtung ein Instrument oder ein Implantat ist.

13. Vorrichtung nach Anspruch 1, wobei der Schieber (21) an der Außenfläche (20) des Rohrs (19) an dem proximalen Endabschnitt (22) des Rohrs angebracht ist.

14. Vorrichtung nach Anspruch 1, wobei die Außenfläche (20) des Rohrs (19) an dem proximalen Endabschnitt (22) des Rohrs ein Gewinde aufweist und der Schieber (23) ein Fenster mit einem zusammenpassenden Gewinde aufweist, und wobei der Schieber mit dem proximalen Endabschnitt des Rohrs in Gewindeeingriff ist.

15. Vorrichtung nach Anspruch 1, wobei die erste Schiene (13) der Kranial-Kaudal-Stange (11) mit der ersten Schiene (9) der Medial-Lateral-Stange (7) mittels einer Bolzen-Schlitz-Verbindung in gleitendem Eingriff ist.

## Revendications

1. Appareil médical comprenant :
un bras (1) qui comporte une partie d'extrémité proximale (3) qui est connectée à un objet stationnaire et une partie d'extrémité distale (5) ;
une barre médio-latérale (7) qui est connectée à la partie d'extrémité distale (5) du bras (1) et qui comporte un premier rail incurvé (9) ;
une barre cranio-caudale (11) qui comporte :
un premier rail incurvé (13) en engagement par coulissement avec le premier rail incurvé (9) de la barre médio-latérale (7) dans une première direction, dans lequel le premier rail incurvé de la barre cranio-caudale et le premier rail incurvé de la barre médio-latérale présentent des formes incurvées se correspondant ; et
un deuxième rail incurvé (15) qui s'étend sensiblement perpendiculairement par rapport au premier rail incurvé (13) de la barre cranio-caudale ;
une construction de canal de travail (17) qui comprend :
un tube (19) qui comporte une surface externe (20), une partie d'extrémité proximale (22) et une partie d'extrémité distale ; et
un coulisseau (21) qui est lié à la surface externe (20) du tube (19) et qui comporte un premier rail incurvé (23) en engagement par coulissement avec le deuxième rail incurvé (15) de la barre cranio-caudale (11), dans lequel le premier rail incurvé du coulisseau et le deuxième rail incurvé de la barre cranio-caudale présentent des formes incurvées se correspondant ; dans lequel :
les formes incurvées se correspondant du premier rail incurvé (13) de la barre cranio-caudale (11) et du premier rail incurvé (9) de la barre médio-latérale (7) permettent l'inclinaison du tube (19) dans un premier plan, de manière à définir un angle d'inclinaison médio-latéral du tube tout en maintenant une localisation de la partie d'extrémité distale du tube ; et dans lequel :
les formes incurvées se correspondant du premier rail incurvé (23) du coulisseau (21) et du deuxième rail incurvé (15) de la barre cranio-caudale (11) permettent l'inclinaison du tube (19) dans un second plan, de manière à définir un angle d'inclinaison cranio-caudal du tube tout en maintenant la localisation de la partie d'extrémité distale du tube.

2. Appareil selon la revendication 1, dans lequel la barre cranio-caudale (11) comporte un bouton de libération (25) pour une liaison libérable par rapport à la barre médio-latérale (7).

3. Appareil selon la revendication 1, dans lequel le coulisseau (21) comporte un mécanisme de libération (27) pour une liaison libérable par rapport à la barre cranio-caudale (11).

4. Appareil selon la revendication 1, dans lequel la barre médio-latérale (7) comporte une première fenêtre (29) en son sein pour une réception par coulissement de la barre cranio-caudale (11).

5. Appareil selon la revendication 1, dans lequel la barre cranio-caudale (11) comporte un troisième rail (31) qui s'étend depuis le premier rail (13) de la barre cranio-caudale (11) dans une direction sensiblement parallèle au deuxième rail (15) de la barre cranio-caudale.

6. Appareil selon la revendication 5, dans lequel le coulisseau (21) comprend en outre un second rail qui est sensiblement parallèle au premier rail (23) du coulisseau, dans lequel le second rail du coulisseau est en engagement par coulissement avec le troisième rail (31) de la barre cranio-caudale (11).

7. Appareil selon la revendication 6, dans lequel le tube (19) est disposé entre les deuxième et troisième rails (15, 31) de la barre cranio-caudale (11) .

8. Appareil selon la revendication 7, dans lequel un quatrième rail (35) connecte les deuxième et troisième rails (15, 31) de la barre cranio-caudale (11) de manière à former une seconde fenêtre (37), et le tube (19) s'étend au travers de la seconde fenêtre.

9. Appareil selon la revendication 1, dans lequel le premier rail (9) de la barre médio-latérale (7) et le premier rail (13) de la barre cranio-caudale (11) comportent des dents se correspondant (39) sur eux.

10. Appareil selon la revendication 1, dans lequel le deuxième rail (15) de la barre cranio-caudale (11) et le premier rail (23) du coulisseau (21) comportent des dents se correspondant (41) sur eux.

11. Appareil selon la revendication 1, comprenant en outre :
un dispositif médical qui est localisé à l'intérieur du tube.

12. Appareil selon la revendication 11, dans lequel le dispositif médical est un instrument ou un implant.

13. Appareil selon la revendication 1, dans lequel le coulisseau (21) est lié à la surface externe (20) du tube (19) au niveau de la partie d'extrémité proximale (22) du tube.

14. Appareil selon la revendication 1, dans lequel la surface externe (20) du tube (19) au niveau de la partie d'extrémité proximale (22) du tube comporte un filet sur elle, le coulisseau (21) comporte une fenêtre qui comporte un filet conjugué sur elle, et le coulisseau est engagé par mise en prise par filetage avec la partie d'extrémité proximale du tube.

15. Appareil selon la revendication 1, dans lequel le premier rail (13) de la barre cranio-caudale (11) est en engagement par coulissement avec le premier rail (9) de la barre médio-latérale (7) en vertu d'une connexion par boulon(s) - fente(s).
